Europäisches Patentamt

(19) European Patent Office

Office européen des brevets

(11) Publication number: **0 448 300 A2**

# (12) EUROPEAN PATENT APPLICATION

(21) Application number: **91302221.6**

(22) Date of filing: **15.03.91**

(51) Int. Cl.⁵: **A61N 1/30, A61K 31/415,**
**A61K 47/22, A61K 31/165,**
**// (A61K31/415, 31:165),**
**(A61K31/165, 31:135)**

(30) Priority: **15.03.90 US 494062**

(43) Date of publication of application:
**25.09.91 Bulletin 91/39**

(84) Designated Contracting States:
**AT BE CH DE DK ES FR GB GR IT LI LU NL SE**

(71) Applicant: **Becton Dickinson and Company**
**One Becton Drive**
**Franklin Lakes New Jersey 07417-1880 (US)**

(72) Inventor: **Sage, Burton H., Jr.**
**8408 Lakewood Drive**
**Raleigh, NC 27613 (US)**
Inventor: **Riviere, James E.**
**5105 Lenoraway Drive**
**Raleigh, NC 27613 (US)**

(74) Representative: **Ruffles, Graham Keith et al**
**MARKS & CLERK 57-60 Lincoln's Inn Fields**
**London WC2A 3LS (GB)**

(54) Method and composition for coiontophoresis.

㊄ Methods for changing the delivery of an active ingredient to the vasculature of an animal by iontophoresis have the steps of adding a vasoactivator to a formulation with an active ingredient, coiontophoresing the vasoactivator and the active ingredient into the skin of an animal and establishing a concentration of the vasoactivator in the skin near the upper capillary loops thereof so that the blood flow is changed therethrough. The vasoactivator effectively changes the blood flow in the upper capillary loops of the vasculature in the skin of an animal. If the vasoactivator is a vasodilator, the blood flow is increased and when the vasoactivator is a vasoconstrictor, the blood flow is decreased. An iontophoretic delivery apparatus for use with a formulation for controlling iontophoretic transdermal administration of an active ingredient into the blood stream of the vasculature of an animal has a donor reservoir and an indifferent reservoir with an electric field therebetween for the flow of ions thereacross. The formulation is associated with the donor reservoir for coiontophoresis along the electric field to the indifferent reservoir. The vasoactivator in the formulation changes the blood flow in the upper capillary loops controlling the introduction of the active ingredient into the blood stream of the animal. A composition for coiontophoretic delivery has a solution of an active ingredient and a vasoactivator in a polar liquid.

EP 0 448 300 A2

# METHOD AND COMPOSITION FOR COIONTOPHORESIS

1. Field of the Invention. This invention relates to the iontophoretic transdermal delivery of an active ingredient such as medication, and more particularly to the coiontophoresis of preferred concentrations of selected vasoactivators to control release of the medication into the vasculature.

2. Background. During iontophoresis, charged compounds are caused to pass from a reservoir attached to the skin of a person or animal into the tissue therebeneath. The process is one wherein the rate of compound flow is a function of the amount of current, the concentration of the compound and the presence of other ions during the iontophoretic episode. Higher concentration of compound, higher levels of current, and lower concentration of other ions will result in greater delivery of the compound.

When the rate of iontophoretic administration of ionic compounds is optimized in terms of concentrations of the desired compound and other ionic moieties present in the system, the efficiency of delivery of the desired compound as measured by the percent of iontophoretic current actually transporting the desired compound is still relatively low compared to rates of administration predicted by Faraday's law. Indeed, save for a few elements of the periodic table, as cited in U.S. 4,744,787 efficiencies are less than ten percent. Thus there is a clear need to find methods of obtaining higher efficiencies and hence higher rates of administration.

It is well known, for example, see in "Transdermal Delivery of Drugs" by Brown & Langer, that the rate limiting barrier for transdermal drug delivery is the stratum corneum. Indeed, there continues to be a large research effort to find methods to reduce or eliminate this rate limiting property of the stratum corneum. An understanding of the barrier properties of skin is presented in the "Walters, Percutaneous Absorption and Transdermal Therapy" article but no mention of iontophoresis appears therein.

The Bellantone et al. article entitled, "Enhanced Percutaneous Absorption via Iontophoresis: I. Evaluation of an In Vitro System and Transport of Model Compounds" shows that iontophoresis can be used in place of other means to enhance drug transport through the epidermal barrier such that the need for chemical penetration enhancers could be obviated or alternatively with enhancers lower drug concentrations or energy required for delivery could be possible.

Another technique believed to enhance the delivery of certain types of active ingredients by iontophoresis is disclosed in European patent application 0 278 473 AI which adds compounds to proteins and other macromolecules to decrease the degree of aggregation of the molecules in the active reservoir. The added compounds have the ability to aid solubility and disassociation of the macromolecules.

It is also well known in the iontophoresis art (for example, see "Iontophoretic Delivery of Nonpeptide Drugs" Formulation Optimum for Maximum Skin Permeability by Sanderson et al.) that the presence of ions other than the desired compound in the donor reservoir formulation reduces the iontophoretic efficiency.

Thus, to those familiar with transdermal drug delivery, and specifically iontophoresis, the inclusion of a second ionic moiety in the reservoir with the desired compound in order to increase the rate of administration would be counterproductive.

In the situation of transdermal delivery where the flux is limited by the stratum corneum, the dermal vasculature, which acts as the means of compound removal from the dermal tissue, has no effect on the administration rate. Regardless of its state of dilation, it is capable of removing all the compound that reaches it. Otherwise, the vasculature would become the rate limiting barrier.

In this situation, placing a vasoactive ingredient in the skin, by any means, near the dermal vasculature for the purpose of altering the blood flow through the dermal vasculature would have no effect. The rate of administration would still be limited by the stratum corneum.

Vasodilators such as tolazoline, nitrates, papaverine, phentolamine, dipyridamole, cyclandelate, isoxsuprine, mecholyl, histamine, and nylidrin are known to dilate blood vessels and use with iontophoresis has been studied. For example, in "Evaluation of the local vasodilator effect of Acetyl-Beta Methylcholin Chloride (Mecholyl) by iontophoresis," by Abramson, et al., a significant increase in blood flow was shown.

Coiontophoresis of vasodilators as a means of enhancing the iontophoretic efficiency of delivery of desired compounds has not been described. Indeed, conventional wisdom regarding both iontophoresis and transdermal drug delivery argues persuasively that the addition of vasodilators ions to the ions of the iontophoretic formulation of the desired compound would be detrimental.

Similarly, vasoconstrictors such as adrenalin, epinephrine and norepinephrine are known to constrict blood vessels. Their use in injectable formulation of local anesthetics for the purpose of extending the duration of local anesthesia is understood.

Coiontophoresis of vasoconstrictors as a means of enhancing the concentration of an active ingredient in the skin has not been demonstrated. Again, conventional wisdom argues that increasing the active ingredient concentration in the skin by the addition of a second ionic moiety would be counterproductive.

## SUMMARY OF THE INVENTION

A composition for iontophoretic delivery of an active ingredient is a part of the preferred invention. The composition may be an active ingredient and a vasoactivator combined in solution in perhaps a polar liquid. The vasoactivator changes the blood flow in the upper capillary loops of the vasculature in the skin of an animal. The preferred polar liquid is water. If the vasoactivator is a vasodilator, the blood flow is increased. If the vasoactivator is a vasoconstrictor, the blood flow is decreased.

An iontophoretic delivery apparatus for use with a formulation for controlling iontophoretic transdermal administration of an active ingredient into the blood stream of an animal is another part of the preferred embodiment herein. The apparatus may have an donor reservoir system and an indifferent reservoir system on the iontophoretic delivery apparatus with an electric field therebetween for the flow of ions thereacross. A diluent as a vehicle for an active ingredient which may have a medication to which the animal should respond and a vasoactivator for changing blood flow in the vasculature of the animal are included. The vasoactivator and the active ingredient are combined in a formulation associated with the donor reservoir system for coiontophoresis along the electric field to the indifferent reservoir system. The vasoactivator changes the blood flow in the upper capillary loops controlling the introduction of the active ingredient into the blood stream of the animal.

A method for changing the delivery of an active ingredient to the vasculature of an animal by iontophoresis may have the steps of adding a vasoactivator to a formulation with an active ingredient, coiontophoresing the vasoactivator and the active ingredient into the skin of the animal and establishing a concentration of the vasoactivator in the skin near the upper capillary loops thereof so that the blood flow is changed therethrough.

A method for changing the delivery of an active ingredient to the vasculature of an animal by iontophoresis may have the steps of adding a vasoactivator to a formulation with an active ingredient and placing the formulation into the donor reservoir of an iontophoretic system. The iontophoretic system is placed on the skin of the animal and activated to transdermally deliver the active ingredient to the vasculature in which blood flow has been changed by coiontophoresis of the active ingredient and vasoactivator.

A method for transdermal iontophoretic delivery of an active ingredient into the upper capillary loops of the vasculature of an animal may include the steps of selecting an active ingredient to be iontophoretically delivered and selecting an appropriate vasoactivator capable of changing the blood flow in the upper capillary loops. Thereafter a formulation of the active ingredient and the vasoactivator is placed in the donor reservoir of the iontophoretic system and the donor reservoir and the indifferent reservoir are placed on the animal into which iontophoresis of the active ingredient is desired. An electric power source in series with the donor reservoir system and the indifferent reservoir system begins current flow in a circuit therebetween causing ions of the active ingredient and the vasoactivator to pass into the skin of the animal. Establishing a concentration of the vasoactivator in the skin of the animal such that a rate of administration of the active ingredient changes with the addition of the vasoactivator over a rate of administration of the active ingredient without the vasoactivator is the final step of this part of the invention.

An alternate method for the transdermal iontophoretic delivery of an active ingredient and a vasoactivator into the upper capillary loops of the vasculature of an animal may have the steps of selecting a vasoactivator with the same ionic polarity as an active ingredient desired to be iontophoretically delivered and determining a ratio of the vasoactivator to the active ingredient which provides a formulation for the changed transdermal iontophoretic delivery into the vasculature of the active ingredient over the iontophoresis of the active ingredient without the vasoactivator. Thereafter the final step of coiontophoresing the active ingredient and vasoactivator into the upper capillary loops of the animal is performed.

The method may further include the additional step of selecting the active ingredient from salt compounds and the additional step of selecting the vasoactivator from salt compounds.

A method for enhancing the delivery of an active ingredient to the skin of an animal by iontophoresis has the steps of adding a vasoconstrictor to a formulation with an active ingredient and coiontophoresing the vasoconstrictor and the active ingredient into the skin of the animal. The next step of establishing a concentration of the vasoconstrictor in the skin near the upper capillary loops thereof decreases the blood flow is decreased therethrough.

Another more specific method for the enhanced transdermal iontophoretic delivery of an active ingredient through the skin of an animal includes the steps of selecting a vasoconstrictor from the group of adrenalin, epinephrine and norepinephrine and including the vasoconstrictor in a formulation of diluent and active ingredient. Further steps may be placing the formulation in the donor reservoir of the iontophoresis system and applying the donor reservoir along with an indifferent reservoir against the surface of the skin of the animal in position relative to one another to iontophorese the active ingredient and the vasoconstrictor into the skin. The electric field between the donor reservoir and the indifferent reservoir coiontophoreses active ingredient and the vasoconstrictor along the electric field into the skin near the upper capillary loops for increasing the concentration of the active ingredient in the skin of the animal by reducing blood flow in the upper capillary loops with the vasoconstrictor.

A method for enhancing the delivery of an active ingredient to the vasculature of an animal by iontophoresis has the steps of adding a vasodilator to a formulation with an active ingredient and coiontophoresing the vasodilator with the active ingredient into the skin of the animal. The next step establishes a concentration of the vasodilator in the skin near the upper capillary loops so that the blood flow increases. The step of establishing a preferred concentration may be performed by determining a range of useful concentrations of the vasodilator with respect to the active ingredient by analyzing the amount of active ingredient iontophoresed.

An additional alternative method for the transdermal iontophoretic delivery of an active ingredient to the upper capillary loops for release into the vasculature of an animal is yet another form of the invention. The method may have the steps of selecting a vasodilator from the group of tolazoline, nitrates, papaverine, phentolamine, dipyridamole, cyclandelate, isoxsuprine, mecholyl, histamine and nylidrin and including the vasodilator in a formulation of a diluent and an active ingredient. Placing the formulation within a donor reservoir of a donor reservoir system and applying the donor reservoir system along with an indifferent reservoir system against the surface of the skin and in position relative to one another for coiontophoretic transdermal delivery of the formulation in the donor reservoir into the skin thereadjacent are further steps of this form of the invention. The next step is establishing an electric field between the donor reservoir system and the indifferent reservoir system so that the electric field enters into and passes through the skin near the upper capillary loops. Finally, the step of transmitting the active ingredient of the formulation along the electric field lines and into the upper capillary loops by maintaining a concentration of vasodilator adequate to increase blood flow through the upper capillary loops but insufficient to reach the vessels therebeneath is performed.

## BRIEF DESCRIPTION OF THE DRAWINGS

Figure 1 is a schematic presentation of the skin, which shows the upper capillary loops of the vasculature of the skin and the lower vessels that feed the upper capillary loops and the shunts connected between the lower vessels to control blood flowing in the upper capillary loops.

Figure 2 is a schematic side elevational representation of an iontophoretic system with the donor and indifferent reservoir systems connected to circuitry for driving the active ingredient and vasoactivator into the skin of an animal.

Figure 3 is a plot of data showing that addition of the vasodilator tolazoline to a concentration of lidocaine enhances the iontophoretic rate of administration of lidocaine.

Figure 4 is a plot of data showing that the concentration of the vasodilator has a range wherein the flux of the active ingredient is enhanced, and a range wherein the flux of the active ingredient is diminished.

Figure 5 is a plot of data showing the concentration of lidocaine versus the depth of penetration after coiontophoresis with the vasoconstrictor norepinephrine.

Figure 6 is a plot of data showing the concentration of lidocaine versus the depth of penetration after iontophores is of lidocaine alone.

Figure 7 is a flow diagram of the steps of the preferred method of iontophoretic transdermal delivery of an active ingredient into the blood stream and the enhancement provided by iontophoresis of a vasodilator.

## DETAILED DESCRIPTION OF THE DRAWINGS

While this invention is satisfied by embodiments in many different forms, there is shown in the drawings and will herein be described in detail a preferred embodiment of the invention with the understanding that the present disclosure is to be considered as exemplary of the principles of the invention and is not intended to limit the invention to the embodiment illustrated. The scope of the invention will be measured by the appended claims and their equivalents.

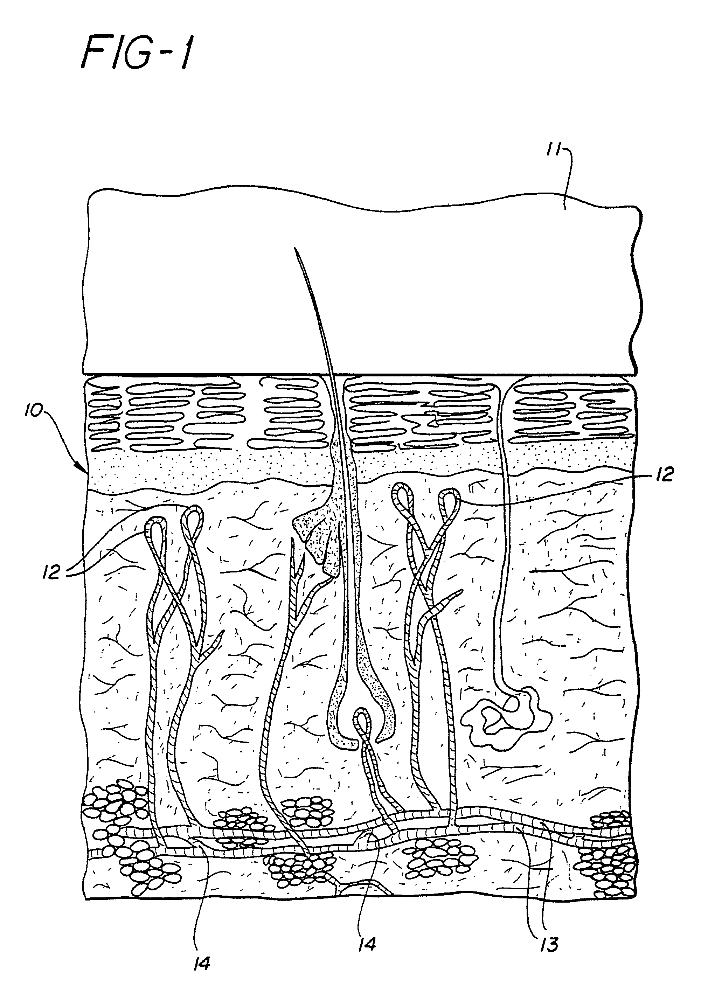

Figure 1 shows a rendering of the skin 10 of an animal, and more specifically the key components of the skin 10 which are important in the transdermal delivery of drugs. As used throughout this specification, the term animal includes humans. Shown in figure 1 are the stratum corneum, the dermis, and more specifically, the blood vessels of the dermis. In systemic drug delivery, the objective is to get the drug from a donor reservoir adjacent to the stratum corneum into the blood stream. In topical drug delivery, the objective is to get the drug from the donor reservoir 11 adjacent to the stratum corneum into the skin below the stratum corneum and avoid removal by the blood stream. Hence the structure of both the stratum corneum and the vasculature is important.

When iontophoresis of an active ingredient is performed, the compound passes through the stratum corneum through the intervening dermal tissue and into the vasculature. In a situation wherein the stratum corneum is the rate-limiting barrier, the blood flow in these upper capillary loops 12 is of little consequence. When the rate of delivery of the compound is enhanced, as in the case of iontophoresis, to the point where the ability of the vasculature to remove the compound is rate limiting, then the blood flow in the upper loops 12 becomes very significant. The amount of drug captured by the vasculature is then dependent upon the blood flow in the upper capillary loops 12 since these are the first blood vessels encountered. The lower or deeper blood vessels 13 are also a part of the vasculature of the skin 10. Shunts bypass the upper capillary loops 12 by connecting the lower blood vessels 13, see Figure 1. Coiontophoresis of a vasoactivator with an active ingredient changes the blood flow thus controlling the rate at which the drug is removed by the vasculature.

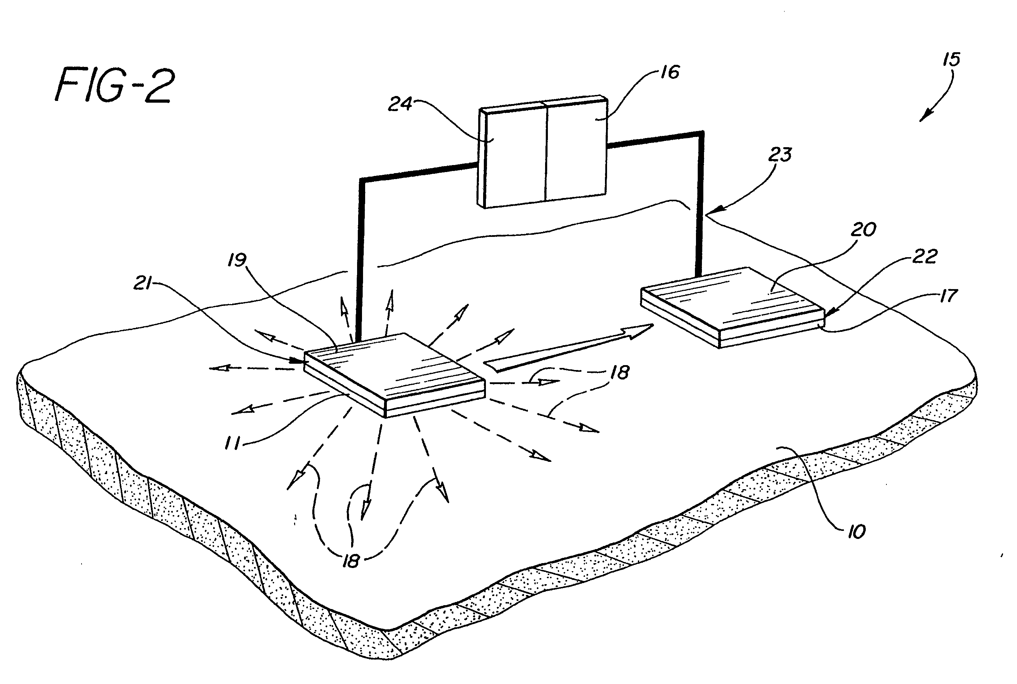

A method for changing the delivery of an active ingredient to the vasculature of an animal by iontophoresis adds a vasoactivator to a formulation with an active ingredient and places the formulation into the donor reservoir 11 of an iontophoretic system 15 (see Figure 2). The iontophoretic system 15, when placed on the skin 10 of the animal, is activated to transdermally deliver the active ingredient to the vasculature in which blood flow is to be changed by coiontophoresis of the vasoactivator.

The preferred iontophoretic system 15 of Figure 2 includes a power source 16 such as a battery connected in series with an indifferent reservoir 17 establishes an electric field 18 between the donor reservoir 11 and the indifferent reservoir 17. A donor electrode 19 connects the power source 16 to the donor reservoir 11. Similarly, an indifferent electrode 20 connects the power source 16 to the indifferent reservoir 17. A donor reservoir system 21 has the donor electrode 19 and the donor reservoir 11 and an indifferent reservoir system has the indifferent electrode 20 and the indifferent reservoir 17. The donor and indifferent reservoir systems 21 and 22 are applied to the skin 10 and are spaced from one another. The iontophoretic system elements 15 may have a plurality of donor and indifferent reservoir systems 21 and 22 between which electric fields 18 pass when the reservoirs 17 and 11 are against the skin 10 of an animal. The donor reservoir 11 contains at least a therapeutic agent and some vehicle or diluent. The indifferent reservoir 17 holds an electrolyte for the purpose of completing the current path established by the electrical field 18 between the donor reservoir 11 and the indifferent reservoir 17.

Specifically, the iontophoretic system 15 may be an iontophoretic delivery apparatus 23 in Figure 2 used with a formulation of an active ingredient for controlled iontophoretic transdermal administration of the active ingredient into the blood stream of an animal. The apparatus has the donor and indifferent reservoir systems 21 and 22 with the electric field 18 therebetween for the flow of ions. A control circuit 24 may be provided to regulate the rate of iontophoresis, if desired. A diluent provides a vehicle for an active ingredient to which the animal should respond and a vasoactivator which changes blood flow in the vasculature of the animal. The diluent, vasoactivator and the active ingredient are in the preferred embodiment combined in a formulation associated with the donor reservoir system 21. The active ingredient and the vasoactivator are coiontophoresed along the electric field 18 to the indifferent reservoir system 22. The vasoactivator changes the blood flow in the upper capillary loops 12, as shown in Figure 1, controlling the rate of introduction of the active ingredient into the blood stream of the animal.

Alternately, the active ingredient and a vasoactivator capable of changing the blood flow in the upper capillary loops 12 are selected and formulated for placement in the donor reservoir 11 of the iontophoretic system 15. The donor reservoir 11 and the indifferent reservoir 17 are placed on the skin 10 of the animal. The electric power source 16, regulated by circuit 24, in series with the donor reservoir system 21 and the indifferent reservoir system 22 provides current flow therebetween causing ions of the active ingredient and the vasoactivator to pass into the skin 10 of the animal. The concentration of the vasoactivator in the skin 10 of the animal is established such that the rate of administration of the active ingredient with the addition of the vasoactivator is greater than the rate of administration of the active ingredient without the vasoactivator.

The preferred composition for altered iontophoretic delivery of the active ingredient is a solution of the active ingredient and the vasoactivator in a polar liquid. The polar liquid is most preferably water. The vasoactivator changes the blood flow in the upper capillary loops 12 of the vasculature in the skin 10 of the animal. If the vasoactivator is a vasodilator, the blood flow is increased. If the vasoactivator is a vasoconstrictor, the blood flow is decreased.

One method for enhancing the delivery of the active ingredient to the vasculature of an animal by iontophoresis has the steps of adding a vasodilator to a formulation with the active ingredient and coiontophoresing the vasodilator and the active ingredient into the skin 10 of the animal. Concentrating the vasodilator in the skin 10 near the upper capillary loops 12 thereof increases blood flow.

As can also be appreciated by reference to Figure 1, any vasodilation effect should be restricted to the areas of the skin 10 which contain the upper capillary loops 12. If the vasodilator reaches and dilates the shunt blood vessels 14 in the skin 10 beneath the upper capillary loops 12, then the blood flow in the upper capillary loops 12 will actually be decreased, not increased. In particular, the shunt blood vessels 14, if dilated act to permit blood to bypass the upper capillary loops 12. Thus, the blood flow through the upper capillary loops 12 is decreased and the delivery of the active ingredient is inhibited. Preferably the concentration of the vasodilator in the skin 10 must be kept relatively high near the upper capillary loops 12 and relatively low near the shunts 14.

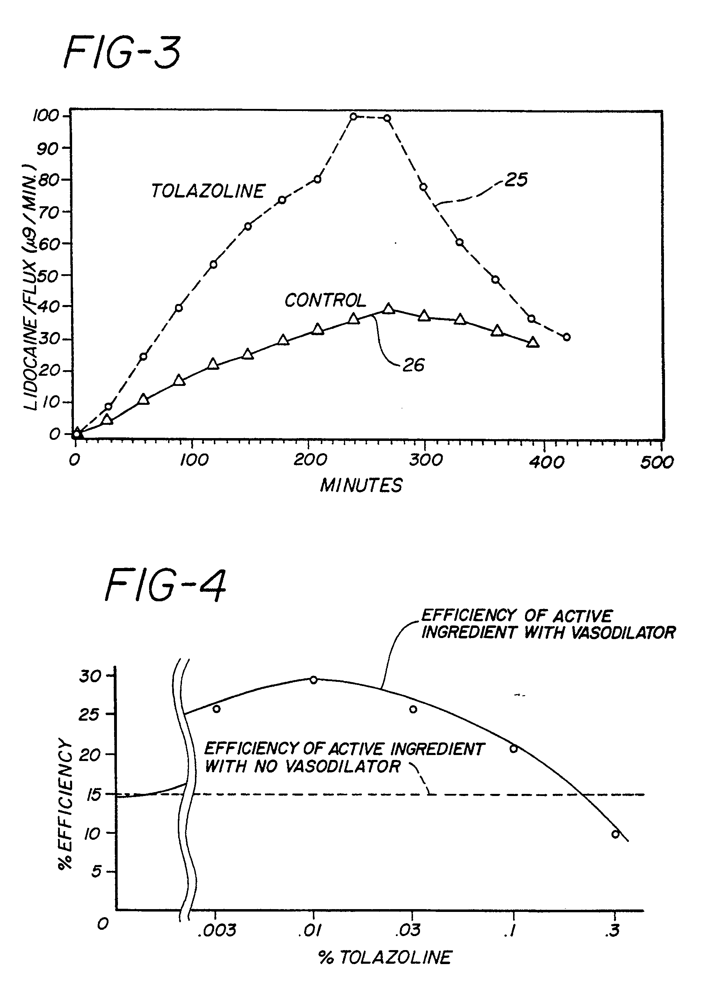

The ability to maintain proper concentration ratio will depend upon the iontophoretic properties of the active ingredient and hence the relative proportions of the vasodilator combined with the active ingredient in the donor reservoir. The data shown in Figure 3, for the vasodilator, tolazoline, and the active ingredient, lidocaine, demonstrates the properties of one form of the invention, but this example is not to limit the scope of the invention to certain ratios or formulations. Figure 3, the upper plot 25, shows the increase in the flux or rate of delivery of lidocaine with the addition of an optimum amount of the vasodilator tolazoline. The lower plot 26 shows iontophoresis of a control without a vasodilator. The improvement in transdermal delivery by the coiontophoresis of the active ingredient, lidocaine, with the vasodilator, tolazoline, is apparent by comparing the upper and lower plots 25 and 26.

Figure 4 shows that there is an optimum concentration of vasodilator when the concentration of the active ingredient is kept constant. With no vasodilator, the efficiency is on the order of 15%. At the optimum, the efficiency is over 30%. As more vasodilator is added, beyond the optimum concentration, the efficiency falls to a level lower than the original level.

One method for enhancing the delivery of an active ingredient to the skin 10 of an animal by iontophoresis includes the steps of adding a vasoconstrictor to a formulation with an active ingredient and coiontophoresing the vasoconstrictor and the active ingredient into the skin 10 of the animal. Concentrating the vasoconstrictor in the skin near the upper capillary loops 12 decreases blood flow therethrough.

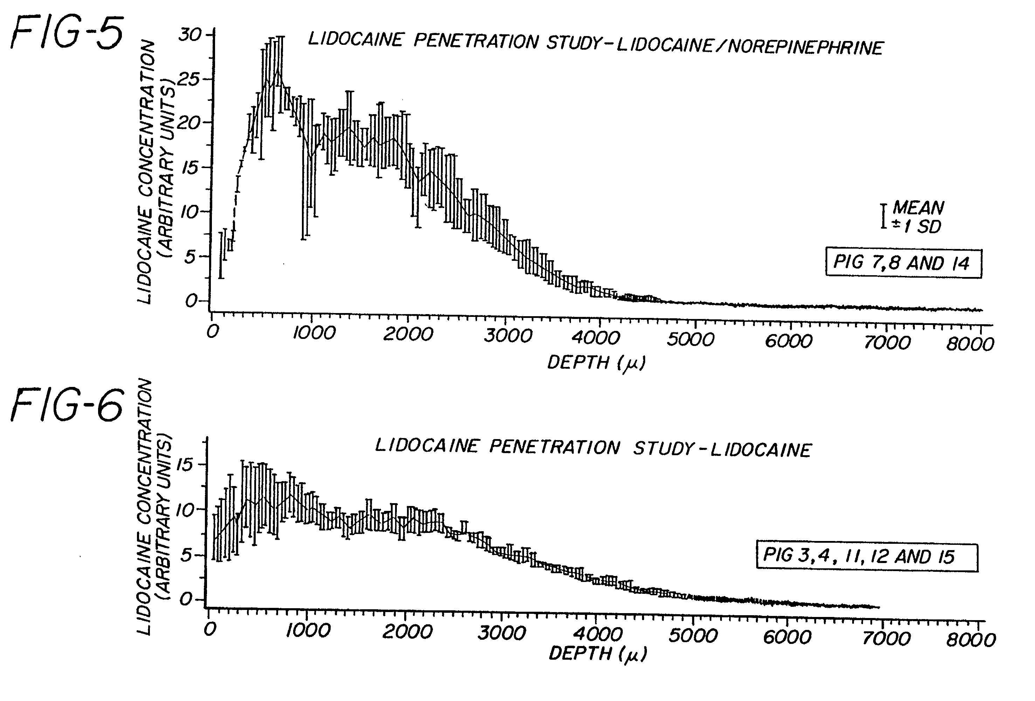

Figure 5 is a plot of data showing that the addition of a vasoconstrictor to a concentration of lidocaine increases the concentration of lidocaine in the tissue near the upper capillary loops 12, and hence reduces the delivery of lidocaine to the blood stream of the animal. Tests were performed using radiolabelled lidocaine in vivo in swine. Figure 6 is a plot of data showing the concentration of lidocaine without any vasoconstrictor versus the depth of penetration.

In these examples, the object of the addition of a vasoconstrictor is to increase the concentration of the active ingredient in the skin 10 beneath the donor reservoir 11 and not in the blood stream or vasculature of the animal. The data shown is the actual concentration of the active ingredient in the skin 10 as measured by counting iontophoresed radiolabelled lidocaine. This differs from the data in Figures 3 and 4 which shows increased concentration of lidocaine in the blood stream after the addition of a vasodilator.

As can be seen in Figure 5, the concentration of lidocaine has been increased in the skin 10 by the addition of the vasoconstrictor, norepinephrine, to the lidocaine formulation in the donor reservoir 11. The coiontophoresis of both lidocaine and norepinephrine restricts blood flow and maintains more lidocaine in the skin 10. Figure 6 shows that the lidocaine without a vasoconstrictor is less concentrated in the skin 10 below the donor reservoir 11. This results because normal blood flow through unconstricted vasculature carries away the lidocaine. For purposes of introducing and maintaining the pain killing properties of lidocaine in the skin 10, coiontophoresis of lidocaine and a vasoconstrictor provides a useful technique.

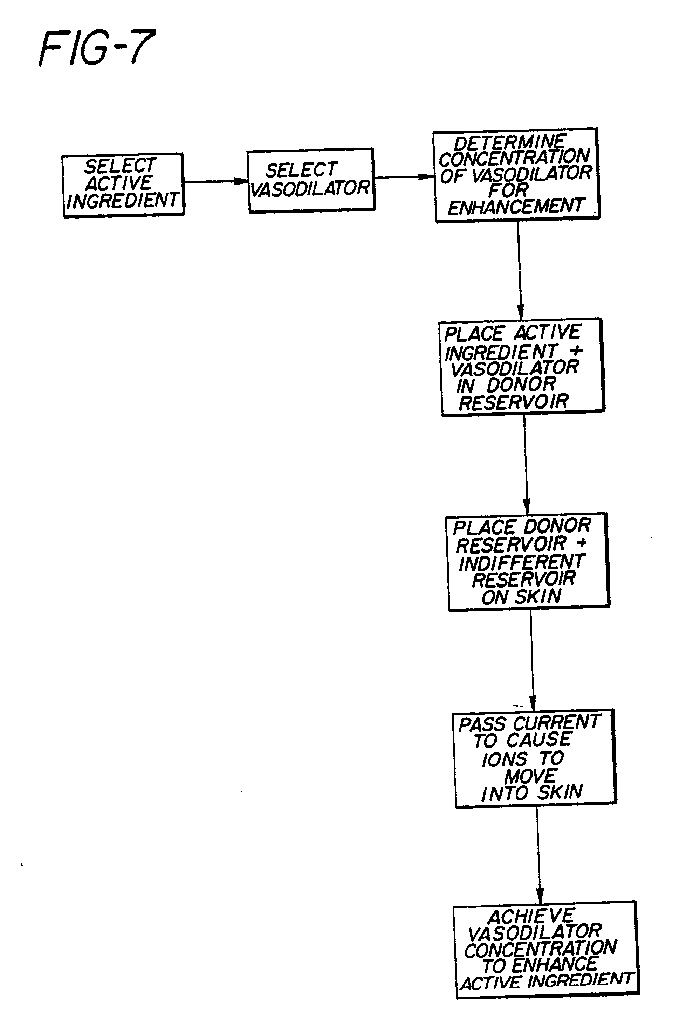

Figure 7 shows a flow diagram of the steps in one form of the method of iontophoretic delivery of the active ingredient into the blood stream of a body, achieved by coiontophoresis, which results in enhanced flux and efficiency. Efficiency is related to either the amount of active ingredient delivered or the amount of energy needed to deliver the active ingredient with and without a vasodilator. A similar flow diagram could be drawn for the method steps used with a vasoconstrictor.

In another method for changing the delivery of the active ingredient to the vasculature of an animal by iontophoresis, the vasoactivator added to a formulation with the active ingredient is coiontophoresed into the skin of the animal. A concentration of the vasoactivator is established in the skin 10 near the upper capillary loops 12 thereof so that the blood flow is changed therethrough. A range of useful concentrations of the vasoactivator with respect to the active ingredient is determined by analyzing the amount of active ingredient iontophoresed.

Also the method may have the steps of picking a vasoactivator with the same ionic polarity as the active ingredient desired to be iontophoresed and determining a ratio of the vasoactivator to the active ingredient which provides an optimum formulation. The method may include the additional steps of selecting the active ingredient and the vasoactivator from salt compounds.

The addition of a vasoactivator to the active ingredient controls the flux or rate of administration of the active ingredient by changing the blood flow in the upper capillary loops 12 of the skin 10 of the animal. This change in blood flow through the upper capillary loops 12 controls the delivery of the active ingredient into the blood stream. If the vasoactivator concentration is adequate to effect the upper capillary loops 12, then the flux of the active ingredient is changed. The concentration or amount of vasoactivator to active ingredient in a formulation or mixture is a function of the particular active ingredient and the vasoactivator used. More specifically, the ease with which the active ingredient may be delivered by iontophoresis is related to the characteristics of the vasoactivator, the active ingredient, and to some extent the iontophoretic system 15.

The concentration of the vasoactivator will effect the delivery flux in two ways. If the vasoactivator is a vasodilator, and there is too much vasodilator, the changed blood flow will occur at the lower or deeper vessels 13 of the skin 10, thus opening shunts 14 which will divert blood flow from the capillary loops 12, and therefore prevent enhancement of active ingredient delivery. The other effect is the result of the introduction of additional ions that compete with the ions of the active ingredient during the coiontophoresis. Consequently, there is an optimum concentration at which the enhancement is maximized. Coiontophoresis of the vasodilator in the formulation of the active ingredient is more efficient than iontophoresing active ingredient without the vasodilator. The addition of vasodilator permits the same flux with a lower power or put another way makes the iontophoretic system 15 capable of greater efficiency.

A particular method has the steps of selecting a vasodilator from the group of tolazoline, nitrates, papaverine, phentolamine, dipyridamole, cyclandelate, isoxsuprine, mecholyl, histamine and nylidrin and including the vasodilator in a formulation of a diluent and an active ingredient. Placing the formulation within the donor reservoir 11 of the donor reservoir system 21 and applying an indifferent reservoir system 22 against the surface of the skin 10 and in position relative to one another for coiontophoretic delivery of the vasodilator and the active ingredient into the skin 10 adjacent to donor reservoir 11 are further steps of this method. Maintaining a concentration of vasodilator adequate to increase blood flow through the upper capillary loops 12 but insufficient to reach the lower vessels 13 and the shunts 14 therebeneath thereby increasing transmission of active ingredient into the upper capillary loops 12 and hence the blood stream of the animal completes this method.

A specific method for the enhanced transdermal iontophoretic delivery of an active ingredient into the skin 10 has the steps of selecting a vasoconstrictor from the group of adrenalin, epinephrine and norepinephrine and including the vasoconstrictor in a formulation of diluent and active ingredient. Placing the formulation in the donor reservoir 11 of donor reservoir system 21 of the iontophoresis system 15 and applying the indifferent reservoir system 22 against the surface of the skin 10 of the animal in position relative to one another to permit coiontophoretic transdermal delivery of the active ingredient and the vasoconstrictor into the skin 10 adjacent the donor reservoir 11 are further steps of this method. The method concludes by establishing the electric field 18 between the donor reservoir 11 and the indifferent reservoir 17 and transmitting the active ingredient and the vasoconstrictor along the electric field 18 into the skin 10 near the upper capillary loops 12 for increasing the concentration of the active ingredient in the skin 10 by reducing blood flow in the upper capillary loops 12 with the vasoconstrictor.

## Claims

1. A composition for iontophoretic delivery of an active ingredient, the composition comprising;
   a polar liquid and an active ingredient as a solution, and
   a vasoactivator dissolved in the solution, the vasoactivator for effectively changing the blood flow in the upper capillary loops of the vasculature in the skin of an animal.

2. A composition according to Claim 1 wherein the vasoactivator is a vasodilator and the blood flow is increased thereby.

3. A composition according to Claim 1 wherein the vasoactivator is a vasoconstrictor and the blood flow is decreased thereby.

4. An iontophoretic delivery apparatus with a formulation used therewith for changing iontophoretic transdermal administration of an active ingredient into the blood stream of an animal comprising:
   a donor reservoir system and an indifferent reservoir system on the iontophoretic delivery apparatus with an electric field therebetween for the flow of ions thereacross;
   a formulation associated with the donor reservoir system for coiontophoresis along the electric field to the indifferent electrode system of the active ingredient therein being a medication to which the animal should respond and a vasoactivator for changing blood flow in the upper capillary loops of the animal combined in a diluent as a vehicle for the active ingredient and the vasoactivator.

5. A method for changing the delivery of an active ingredient to the vasculature of an animal by iontophoresis comprising the following steps:
   mixing a vasoactivator to a formulation with an active ingredient;
   placing the formulation into a donor reservoir of an iontophoretic system;
   placing the iontophoretic system on the skin of the animal;
   activating the iontophoresis system to transdermally deliver the active ingredient and the vasoactivator to the vasculature, and
   changing the delivery of the active ingredient with the action of the vasoactivator on the vasculature.

6. A method for changing the delivery of an active ingredient to the vasculature of an animal by iontophoresis comprising the following steps;
   mixing a vasoactivator to a formulation with an active ingredient;

coiontophoresing the vasoactivator and the active ingredient into the skin of the animal, and

establishing a concentration of the vasoactivator in the skin near the upper capillary loops thereof so that the blood flow is changed therethrough.

7. A method for the transdermal iontophoretic delivery of an active ingredient into the upper capillary loops of the vasculature of an animal including the following steps:

selecting a vasoactivator with the same ionic polarity as an active ingredient desired to be iontophoretically delivered;

determining a ratio of the vasoactivator to the active ingredient which provides a formulation for the changed transdermal iontophoretic delivery into the vasculature of the active ingredient over the iontophoresis of the active ingredient without the vasoactivator, and

coiontophoresing the active ingredient and vasoactivator into the upper capillary loops of the animal.

8. A method for the transdermal iontophoretic delivery of an active ingredient to the upper capillary loops for release into the vasculature of an animal including the following steps:

selecting a vasodilator from the group of tolazoline, nitrates, papaverine, phentolamine, dipyridamole, cyclandelate, isoxsuprine, mecholyl, histamine and nylidrin;

including the vasodilator in a formulation of a diluent and an active ingredient;

placing the formulation in a donor reservoir of an iontophoresis system;

applying the donor reservoir along with an indifferent reservoir against the surface of the skin and in position relative to one another for coiontophoretic transdermal delivery of the active ingredient and the vasodilator;

establishing an electric field between the donor reservoir and the indifferent reservoir wherein the electric field enters into and passes through the skin near the upper capillary loops;

transmitting the active ingredient and the vasodilator along the electric field into skin near the upper capillary loops, and increasing the delivery of the active ingredient by maintaining a concentration of vasodilator adequate to increase blood flow through the upper capillary loops.

9. A method for enhancing the delivery of an active ingredient to the vasculature of an animal by iontophoresis comprising the following steps:

mixing a vasodilator to a formulation with and active ingredient;

coiontophoresing the vasodilator and the active ingredient into the skin of the animal; and

establishing a concentration of the vasodilator in the skin thereof, so that the blood flow is increased therethrough.

10. A method for enhancing the delivery of an active ingredient to the skin of an animal by iontophoresis comprising the following steps:

mixing a vasoconstrictor to a formulation with an active ingredient;

coiontophoresing the vasoconstrictor and the active ingredient into the skin of an animal, and

establishing a concentration of the vasoconstrictor in the skin thereof, so that the blood flow is decreased therethrough.

# FIG-1

FIG-2

## FIG-3

## FIG-4

FIG-5

LIDOCAINE PENETRATION STUDY-LIDOCAINE/NOREPINEPHRINE

LIDOCAINE CONCENTRATION (ARBITRARY UNITS)

MEAN ± 1 SD

PIG 7,8 AND 14

DEPTH (μ)

FIG-6

LIDOCAINE PENETRATION STUDY-LIDOCAINE

LIDOCAINE CONCENTRATION (ARBITRARY UNITS)

PIG 3,4,11,12 AND 15

DEPTH (μ)

EP 0 448 300 A2

# FIG-7

```
┌──────────────┐     ┌──────────────┐     ┌──────────────────┐
│   SELECT     │     │   SELECT     │     │   DETERMINE      │
│   ACTIVE     │ ──▶ │  VASODILATOR │ ──▶ │  CONCENTRATION   │
│  INGREDIENT  │     │              │     │ OF VASODILATOR   │
└──────────────┘     └──────────────┘     │      FOR         │
                                          │  ENHANCEMENT     │
                                          └──────────────────┘
                                                   │
                                                   ▼
                                          ┌──────────────────┐
                                          │  PLACE ACTIVE    │
                                          │ INGREDIENT +     │
                                          │  VASODILATOR     │
                                          │  IN DONOR        │
                                          │  RESERVOIR       │
                                          └──────────────────┘
                                                   │
                                                   ▼
                                          ┌──────────────────┐
                                          │  PLACE DONOR     │
                                          │ RESERVOIR +      │
                                          │  INDIFFERENT     │
                                          │  RESERVOIR       │
                                          │   ON SKIN        │
                                          └──────────────────┘
                                                   │
                                                   ▼
                                          ┌──────────────────┐
                                          │ PASS CURRENT     │
                                          │  TO CAUSE        │
                                          │   IONS TO        │
                                          │    MOVE          │
                                          │  INTO SKIN       │
                                          └──────────────────┘
                                                   │
                                                   ▼
                                          ┌──────────────────┐
                                          │   ACHIEVE        │
                                          │  VASODILATOR     │
                                          │ CONCENTRATION    │
                                          │  TO ENHANCE      │
                                          │ ACTIVE INGREDIENT│
                                          └──────────────────┘
```